(19) Europäisches Patentamt
European Patent Office
Office européen des brevets

(11) **EP 4 544 918 A1**

(12) **EUROPEAN PATENT APPLICATION**
published in accordance with Art. 153(4) EPC

(43) Date of publication:
**30.04.2025 Bulletin 2025/18**

(21) Application number: **23827224.9**

(22) Date of filing: **21.06.2023**

(51) International Patent Classification (IPC):
*A23G 9/36* (2006.01)      *A23D 9/00* (2006.01)
*A23G 9/34* (2006.01)      *A23G 9/42* (2006.01)
*A23L 9/20* (2016.01)      *A23L 29/212* (2016.01)
*C12N 9/20* (2006.01)      *C12N 9/26* (2006.01)

(52) Cooperative Patent Classification (CPC):
A23D 9/00; A23G 9/34; A23G 9/36; A23G 9/42;
A23L 9/20; A23L 29/212; C12N 9/20; C12N 9/2408

(86) International application number:
**PCT/JP2023/022885**

(87) International publication number:
**WO 2023/249041 (28.12.2023 Gazette 2023/52)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **22.06.2022 JP 2022100064**

(71) Applicants:
• **Amano Enzyme Inc.**
  **Nagoya-shi**
  **Aichi 460-8630 (JP)**

• **Amano Enzyme USA Co., Ltd.**
  **Elgin, IL 60124 (US)**

(72) Inventors:
• **HENRY Monica**
  **Elgin, Illinois 60124 (US)**
• **OKUDA Keita**
  **Kakamigahara-shi, Gifu 509-0109 (JP)**

(74) Representative: **Mullholland, Lewis Paul**
**WP Thompson**
**1 Mann Island**
**Liverpool L3 1BP (GB)**

(54) **ENZYME PREPARATION FOR IMPROVING SHAPE RETAINABILITY**

(57)      [Summary]

To provide a technique for improving the shape-retainability of a plant-base ice cream.

The technique provides an enzyme preparation for improving the shape-retainability of a plant-base ice cream, which contains one or more enzymes selected from the group consisting of maltotriose-producing amylase, lipase, and β-amylase, and a plant-base ice cream using the enzyme preparation for improving the shape-retainability. The technique also provides a method for producing a plant-base ice cream and a method for improving the shape-retainability of a plant-base ice cream, which include one or more steps selected from a maltotriose-producing amylase action step of allowing maltotriose-producing amylase to act on a carbohydrate-containing plant-base material, a lipase action step of allowing lipase to act on a fat-containing plant-base material, and a β-amylase action step of allowing β-amylase to act.

EP 4 544 918 A1

**Description**

Technical Field

The present invention relates to an enzyme preparation for improving the shape-retainability of a plant-base ice cream. More specifically, the present invention relates to an enzyme preparation for improving the shape-retainability for improving the quality of a plant-base ice cream, a plant-base ice cream, a method for producing a plant-base ice cream, and a method for improving the shape-retainability of a plant-base ice cream.

Background Art

**In** recent years, the food market has seen an expansion of the milk substitute and meat substitute markets due to sustainability and health consciousness. Plant-base ice creams occupy an important category in the milk substitute market and continue to be a field that attracts attention. It is standard to manufacture it by mixing plant-base milk with a protein source and a lipid source as needed and emulsifying it.

Under these circumstances, techniques for improving the quality of a plant-base ice cream are being developed. For example, Patent Literature 1 discloses a production technique for reducing the amount of plant-base protein used in order to improve the flavor of a plant-base ice cream that minimizes the amount of animal-origin ingredients such as milk ingredients.

Citation List

Patent Literature

Patent Literature 1: U.S. Patent Publication No. 2018/0184684

Summary of Invention

Technical Problem

As mentioned above, plant-base foods and beverages have been attracting attention in recent years due to the growing health consciousness, but a plant-base ice cream has a fundamentally different composition from a dairy ice cream, so there is still room for improvement to obtain satisfactory properties. For example, shape-retainability is related to the stability of the product, and is an important property that should be further improved in order to prevent a plant-base ice cream from melting or deforming due to heat shock during storage.

Therefore, the main objective of this technique is to provide a technique for improving the shape-retainability of a plant-base ice cream.

Solution to Problem

As a result of intensive research into techniques for improving the shape-retainability of a plant-base ice cream, the inventors of the present application discovered that the shape-retainability of a plant-base ice cream can be improved by allowing maltotriose-producing amylase, lipase, or β-amylase to act on plant-base materials, and thus completed the present technique.

That is, the present technique first provides an enzyme preparation for improving the shape-retainability of a plant-base ice cream, which contains one or more enzymes selected from the group consisting of maltotriose-producing amylase, lipase, and β-amylase.

Next, the present technique provides a plant-base ice cream in which the enzyme preparation for improving the shape-retainability according to the present technique is used.

The present technique further provides a method for producing a plant-base ice cream and a method for improving the shape-retainability of a plant-base ice cream, which include one or more steps selected from a maltotriose-producing amylase action step in which maltotriose-producing amylase is allowed to act on a carbohydrate-containing plant-base material, a lipase action step in which lipase is allowed to act on a fat-containing plant-base material, and a β-amylase action step in which β-amylase is allowed to act on a carbohydrate-containing plant-base material.

Advantageous Effects of Invention

According to the present technique, it is possible to improve the shape-retainability of a plant-base ice cream, and to provide a plant-base ice cream that is highly stable during storage and does not melt easily.

Description of Embodiments

A preferred embodiment for carrying out the present invention will be described below. Note that the embodiment described below is an example of a typical embodiment of the present invention, and the scope of the present invention is not to be construed narrowly by this embodiment.

1. Enzyme preparation for improving the shape-retainability

The enzyme preparation for improving the shape-retainability according to the present technique contains one or more enzymes selected from the group consisting of maltotriose-producing amylase, lipase, and β-amylase as active ingredients. In addition, it is possible to contain α-amylase, monoglyceride, diglyceride, other ingredients, etc., as necessary. Each ingredient will be described in detail below.

(1) Maltotriose-producing amylase

The maltotriose-producing amylase that can be used in the present technique is an enzyme that acts on starch and has an activity of mainly producing maltotriose. The maltotriose-producing amylase that can be used in the present technique may also be an enzyme that has other functions as long as it has the activity of producing maltotriose.
In this technique, the shape-retainability of the produced plant-base ice cream can be improved by allowing maltotriose-producing amylase to act on plant-base materials.
The origin of the maltotriose-producing amylase that can be used in the present technique is not particularly limited, and examples thereof include maltotriose-producing amylases derived from organisms of the genus Streptomyces (Streptomyces griseus, etc.), the genus Bacillus (Bacillus subtilis, etc.), the genus Microbacterium (Microbacterium sp., etc.) disclosed in JP-A-03-251173, and the genus Cellulosimicrobium (Cellulosimicrobium) disclosed in WO2020/090734. One type of these maltotriose-producing amylases may be used alone, or multiple types may be used in combination. Among these maltotriose-producing amylases, from the viewpoint of further improving the shape-retainability of a plant-base ice cream, it is preferable to select a maltotriose-producing amylase derived from an organism of the genus Microbacterium, and it is more preferable to select a maltotriose-producing amylase derived from Microbacterium sp. organisms.
Here, "maltotriose-producing amylase derived from Microbacterium sp. organisms" means maltotriose-producing amylase produced by a microorganism (whether a wildtype stain or a mutant strain) classified as Microbacterium sp., or maltotriose-producing amylase obtained by a genetic engineering technique using a maltotriose-producing amylase gene. Therefore, a recombinant produced by a host microorganism into which a maltotriose-producing amylase gene (or a modified gene of said gene) obtained from Microbacterium sp. organisms has been introduced also falls under "maltotriose-producing amylase derived from Microbacterium sp. organisms".
The maltotriose-producing amylase used in the present technique can be prepared from a culture solution of a microorganism from which the maltotriose-producing amylase is derived. A specific preparation method includes a method of recovering the maltotriose-producing amylase from the culture solution or cells of the microorganism. For example, when a maltotriose-producing amylase-secreting microorganism is used, the cells can be recovered from the culture solution in advance by filtration, centrifugation, or the like as necessary, and then the enzyme can be separated and/or purified. In addition, when a maltotriose-producing amylase-non-secreting microorganism is used, the cells can be recovered from the culture solution in advance as necessary, and then the cells can be crushed by pressure treatment, ultrasonic treatment, or the like to expose an enzyme, and then the enzyme can be separated and/or purified. As the method for separating and/or purifying the enzyme, a known protein separation and/or purification method can be used without any particular limitation, and examples thereof include centrifugation, UF concentration, salting out, and various chromatography methods using ion exchange resins, etc. The separated and/or purified enzyme can be powdered by a drying method such as freeze-drying or drying under reduced pressure, or can be powdered by using an appropriate excipient and/or drying aid in the drying method. Further, the separated and/or purified enzyme can also be liquefied by adding an appropriate additive and sterilizing by filtration.
In the present technique, a commercially available product can be used as the maltotriose-producing amylase, and a preferred example of a commercially available product is a maltotriose-producing amylase manufactured by Amano Enzyme Inc.
The content of the maltotriose-producing amylase in the enzyme preparation for improving the shape-retainability according to the present technique can be freely set as long as it does not impair the effect of the present technique. The content of the maltotriose-producing amylase can be set to, for example, 0.05 U or more per 1 g of the carbohydrate-containing plant-base material on which the maltotriose-producing amylase is to act, and from the viewpoint of further improving the shape-retainability, the content can be set to preferably 0.3 U or more, more preferably 0.6 U or more, even more preferably 3 U or more, and even more preferably 6 U or more.

In addition, the content of the maltotriose-producing amylase can be set to, for example, 0.08 U or more per 1 g of polysaccharide (preferably starch) on which the maltotriose-producing amylase is to act, and from the viewpoint of further improving shape-retainability, it can be set to preferably 0.5 U or more, more preferably 1 U or more, even more preferably 5 U or more, and even more preferably 10 U or more.

The upper limit of the content of the maltotriose-producing amylase is not particularly limited as long as it does not impair the effects of the present technique, but can be set to, for example, 250 U or less, 50 U or less, 25 U or less, 20 U or less, 15 U or less, or 10 U or less per 1 g of the carbohydrate-containing plant-base material on which the maltotriose-producing amylase is acted.

In addition, the upper limit of the content of the maltotriose-producing amylase can be set to, for example, 400 U or less, 80 U or less, 40 U or less, 30 U or less, 20 U or less, or 15 U or less per 1 g of polysaccharide (preferably starch) on which the maltotriose-producing amylase is to act.

In the present technique, the activity of the maltotriose-producing amylase is a value defined by the following method.

[Definition of maltotriose-producing amylase activity]

An appropriate amount of enzyme is added to 0.5 ml of 2% soluble starch dissolved in 0.1 M phosphate buffer (pH 7.0), and the reaction is carried out at 40°C in a total volume of 1.0 ml, and the amount of maltotriose and other reducing sugars produced is quantified by the Somogyi-Nelson method. The amount of enzyme that produces reducing sugars equivalent to 1 micromole of glucose per minute under this condition is defined as 1 unit (1 U).

(2) Lipase

The lipase that can be used in the present technique is an enzyme that has the activity of hydrolyzing triglycerides to produce diglycerides, monoglycerides, and fatty acids. The lipase that can be used in the present technique may also be an enzyme that has other functions as long as it has the activity of hydrolyzing triglycerides to produce diglycerides, monoglycerides, and fatty acids.

In this technique, by allowing lipase to act on plant-base ingredients, the shape-retainability of the produced plant-base ice cream can be improved, its stability during storage can be increased, and it can be made less likely to melt.

The origin of the lipase that can be used in the present technique is not particularly limited, but examples thereof include lipases derived from organisms of the genus Aspergillus, the genus Candida, the genus Rhizopus, the genus Mucor, and the genus Penicillium. One type of these lipases may be used alone, or multiple types may be used in combination. Among these lipases, it is preferable to select lipases derived from an organism of the genus Penicillium, and more preferable to select lipases derived from Penicillium roqueforti organisms, from the viewpoint of further improving the shape-retainability of a plant-base ice cream.

Here, "lipase derived from Penicillium roqueforti organisms" means lipase produced by a microorganism (whether a wildtype stain or a mutant strain) classified as Penicillium roqueforti, or lipase obtained by genetic engineering techniques using a lipase gene. Therefore, a recombinant produced by a host microorganism into which a lipase gene (or a modified gene of said gene) obtained from Penicillium roqueforti organism has been introduced also falls under "lipase derived from Penicillium roqueforti organisms".

The lipase used in the present technique can be prepared from a culture solution of the microorganism from which the lipase is derived. A specific preparation method includes a method of recovering lipase from the culture solution or cells of the microorganism. For example, when a lipase-secreting microorganism is used, the cells can be recovered from the culture solution in advance by filtration, centrifugation, or the like as necessary, and then the enzyme can be separated and/or purified. When a lipase-non-secreting microorganism is used, the cells can be recovered from the culture solution in advance as necessary, and then the cells can be crushed by pressure treatment, ultrasonic treatment, or the like to expose an enzyme, and then the enzyme can be separated and/or purified. As a method for separating and/or purifying the enzyme, a known protein separation and/or purification method can be used without any particular limitation, and examples of the method include centrifugation, UF concentration, salting out, and various chromatography methods using ion exchange resins, etc. The separated and/or purified enzyme can be powdered by a drying method such as freeze-drying or vacuum drying, and can also be powdered using an appropriate excipient and/or drying aid in the drying method. In addition, the separated and/or purified enzyme can be liquefied by adding appropriate additives and sterilizing by filtration.

In the present technique, a commercially available product can also be used as the lipase, and a preferred example of a commercially available product is lipase derived from an organism of the genus Penicillium manufactured by Amano Enzyme Inc.

The content of lipase in the enzyme preparation for improving the shape-retainability according to the present technique can be freely set as long as it does not impair the effect of the present technique. The content of lipase can be set to, for example, 0.01 U or more per 1 g of the fat-containing plant-base material on which the lipase is to act, and from the viewpoint of further improving the shape-retainability, it can be set to preferably 0.1 U or more, more preferably 0.4 U or more, even more preferably 1 U or more, and even more preferably 2 U or more.

In addition, the content of lipase can be set to, for example, 0.09 U or more per 1 g of the fat on which the lipase is to act, and from the viewpoint of further improving shape-retainability, the content can be set to preferably 0.4 U or more, more preferably 0.9 U or more, even more preferably 1 U or more, and even more preferably 2 U or more.

The upper limit of the lipase content is not particularly limited as long as it does not impair the effects of the present technique, but can be set to, for example, 100 U or less, 20 U or less, 15 U or less, 10 U or less, 5 U or less, or 3 U or less per 1 g of the fat-containing plant-base material on which the lipase is to act.

In addition, the upper limit of the lipase content can be set to, for example, 100 U or less, 20 U or less, 15 U or less, 10 U or less, 5 U or less, or 3 U or less per 1 g of the fat on which the lipase is to act.

In the present technique, the activity of lipase is a value defined by the following method.

[Definition of Lipase Activity]

Forty five grams (45 g) of olive oil is mixed with 150 mL of an emulsion (18.5 g/L polyvinyl alcohol (completely saponified, saponification degree 98.8±0.2), 1.5 g/L polyvinyl alcohol (partially saponified, saponification degree 88.8 ±1.0)) and emulsified using a homogenizer to prepare a substrate solution. One milliliter (1 mL) of a lipase enzyme solution is added to 5mL of the substrate solution and 4 mL of 0.1 mol/L phosphate buffer (pH 7.0) and reacted at 37°C, and after 30 minutes, 10 mL of an ethanol-acetone mixture is added to stop the enzyme reaction. Next, 10 mL of a 0.05 mol/L sodium hydroxide solution and 10 mL of an ethanol-acetone mixture are added, and the mixture is titrated with 0.05 mol/L hydrochloric acid to pH 10. The amount of enzyme that increases 1 $\mu$mol of fatty acid per minute is defined as 1 unit (1U).

(3) $\beta$-Amylase

The $\beta$-amylase that can be used in the present technique is an exo-type enzyme that sequentially degrades $\alpha$-1,4 glucosidic bonds in maltose units from the nonreducing end of starch. The $\beta$-amylase that can be used in the present technique may be an enzyme that further has other functions as long as it has the above-mentioned $\beta$-amylase activity.

In this technique, the shape-retainability of the produced plant-base ice cream can be improved by allowing $\beta$-amylase to act on the plant-base material.

The origin of the $\beta$-amylase that can be used in the present technique is not particularly limited, and examples thereof include $\beta$-amylases derived from organisms of the genus Bacillus (Bacillus flexus, Bacillus megaterium, Bacillus polymyxa, Bacillus circulans, etc.), the genus Pseudomonas, and the genus Streptomyces. One type of these $\beta$-amylases may be used alone, or multiple types may be used in combination. Among these $\beta$-amylases, from the viewpoint of further improving the shape-retainability of a plant-base ice cream, it is preferable to select $\beta$-amylase derived from organisms of the genus Bacillus, and it is more preferable to select $\beta$-amylase derived from Bacillus flexus organisms.

Here, "$\beta$-amylase derived from Bacillus flexus organisms" refers to $\beta$-amylase produced by a microorganism (whether a wildtype stain or a mutant strain) classified as Bacillus flexus, or $\beta$-amylase obtained by a genetic engineering technique using a $\beta$-amylase gene. Therefore, a recombinant produced by a host microorganism into which a $\beta$-amylase gene (or a modified gene of said gene) obtained from a Bacillus flexus organism has been introduced also falls under "$\beta$-amylase derived from Bacillus flexus organisms."

The $\beta$-amylase used in the present technique can be prepared from a culture solution of a microorganism from which the above-mentioned $\beta$-amylase is derived. Specific preparation methods include a method of recovering $\beta$-amylase from the culture solution or cells of the above-mentioned microorganism. For example, when a $\beta$-amylase-secreting microorganism is used, the cells can be recovered from the culture solution in advance by filtration, centrifugation, or the like as necessary, and then the enzyme can be separated and/or purified. When a $\beta$-amylase non-secreting microorganism is used, the cells can be recovered from the culture solution in advance as necessary, and then the cells can be crushed by pressure treatment, ultrasonic treatment, or the like to expose an enzyme, and then the enzyme can be separated and/or purified. As a method for separating and/or purifying the enzyme, a known protein separation and/or purification method can be used without any particular limitation, and examples of the method include centrifugation, UF concentration, salting out, various chromatography methods using ion exchange resins, and the like. The separated and/or purified enzyme can be powdered by a drying method such as freeze-drying or vacuum drying, and can also be powdered using an appropriate excipient and/or drying aid in the drying method. In addition, the separated and/or purified enzyme can be liquefied by adding appropriate additives and sterilizing by filtration.

In addition, the present technique is not limited to the above-mentioned $\beta$-amylase derived from microorganisms, and can also use $\beta$-amylase derived from plants. Examples of the $\beta$-amylase include those derived from plants such as soybean, wheat, barley, etc.

In the present technique, commercially available product can also be used as the β-amylase, and a preferred example of a commercially available product is β-amylase derived from Bacillus flexus-derived organisms manufactured by Amano Enzyme Inc.

The content of β-amylase in the enzyme preparation for improving the shape-retainability according to the present technique can be freely set as long as it does not impair the effect of the present technique. The content of β-amylase can be set to, for example, 0.01 U or more per 1 g of the carbohydrate-containing plant-base material on which the β-amylase is to act, and from the viewpoint of further improving shape-retainability, the content can be set to preferably 0.10 U or more, more preferably 0.50 U or more, even more preferably 1.0 U or more, and even more preferably 1.5 U or more.

In addition, the content of β-amylase can be set, for example, to 0.016 U or more per 1 g of polysaccharide (preferably starch) on which the β-amylase is to act, and from the viewpoint of further improving shape-retainability, the content can be set preferably to 0.16 U or more, more preferably to 0.80 U or more, even more preferably to 1.6 U or more, and even more preferably to 2.4 U or more.

The upper limit of the β-amylase content is not particularly limited as long as it does not impair the effects of the present technique, but can be set, for example, to 100 U or less, 50 U or less, 10 U or less, preferably 7.5 U or less, more preferably 4.0 U or less, and even more preferably 2.5 U or less per 1 g of the carbohydrate-containing plant-base material on which the β-amylase is to act.

In addition, the upper limit of the β-amylase content can be set to, for example, 160 U or less, 80 U or less, 16 U or less, preferably 12 U or less, more preferably 6.4 U or less, and even more preferably 4.0 U or less per 1 g of polysaccharide (preferably starch) on which the β-amylase is to act.

In the present technique, the β-amylase activity is a value defined by the following method.

[Definition of β-amylase activity]

Using potato starch as a substrate, the amount of enzyme that brings about an increase in reducing ability equivalent to 1 mg of glucose per minute is defined as 1 unit (1 U).

(4) α-Amylase

In the present technique, α-amylase can be used. The α-amylase that can be used in the present technique is an enzyme that acts on starch and mainly hydrolyzes α-1,4 glycosidic bonds.

The origin of α-amylase that can be used in the present technique is not particularly limited, and examples thereof include α-amylases derived from organisms of the genus Aspergillus (e.g., Aspergillus oryzae, Aspergillus niger, etc.) and the genus Bacillus (e.g., Bacillus amyloliquefaciens, Bacillus subtilis, Bacillus licheniformis, etc.), preferably α-amylases derived from organisms of the genus Bacillus, and more preferably α-amylases derived from Bacillus amyloliquefaciens organisms.

The α-amylase that can be used in the present technique can be prepared from the culture solution of the microorganism from which the above-mentioned α-amylase is derived. Specifically, the α-amylase can be easily prepared by culturing an α-amylase-producing bacterium and isolating the α-amylase by a known means, or by a method using a gene recombination technique.

In the present technique, commercially available product can also be used as the α-amylase, and a preferred example of a commercially available product is α-amylase (derived from Bacillus amyloliquefaciens organism) manufactured by Amano Enzyme Inc.

The content of α-amylase in the enzyme preparation for improving the shape-retainability according to the present technique can be freely set as long as it does not impair the effects of the present technique. The content of α-amylase can be set to, for example, 0.5 U or more per 1 g of the carbohydrate-containing plant-base material on which the α-amylase is to act, and from the viewpoint of further improving shape-retainability, the content can be set to preferably 2 U or more, more preferably 4 U or more, even more preferably 7 U or more, and even more preferably 10 U or more.

In addition, the content of α-amylase can be set to, for example, 0.8 U or more per 1 g of polysaccharide (preferably starch) on which the α-amylase is to act, and from the viewpoint of further improving shape-retainability, the content can be set to preferably 3 U or more, more preferably 6 U or more, even more preferably 10 U or more, and even more preferably 15 U or more.

The upper limit of the α-amylase content is not particularly limited as long as it does not impair the effects of the present technique, but can be set, for example, to 500 U or less, 100 U or less, 50 U or less, preferably 30 U or less, more preferably 20 U or less, and even more preferably 15 U or less per 1 g of the carbohydrate-containing plant-base material on which the α-amylase is to act.

In addition, the upper limit of the α-amylase content can be set to, for example, 800 U or less, 160 U or less, 80 U or less, preferably 40 U or less, more preferably 30 U or less, and even more preferably 20 U or less per 1 g of polysaccharide (preferably starch) on which the α-amylase is to act.

In the present technique, the activity of α-amylase is a value defined by the following method.

[Definition of α-amylase activity]

The amylase activity is measured by iodine reaction using potato starch as a substrate. In other words, the amylase activity is measured by carrying out an enzyme reaction using potato starch as a substrate in a conventional manner, and the amount of enzyme that reduces the color produced by the iodine reaction by 10% in 1 minute is defined as 1 unit (1 U).

In the present technique, when maltotriose-producing amylase and α-amylase are used in combination, the content ratio is determined based on the above-mentioned contents of each enzyme, but from the viewpoint of further enhancing the effect, the content of maltotriose-producing amylase per 1 U of α-amylase can be set to preferably 0.001 U or more, more preferably 0.01 U or more, even more preferably 0.05 U or more, even more preferably 0.08 U or more, even more preferably 0.1 U or more, even more preferably 0.2 U or more, and even more preferably 0.5 U or more.

The upper limit of the range of the content of maltotriose-producing amylase per 1 U of α-amylase can be set, for example, to 100 U or less, and from the viewpoint of further enhancing the shape-retainability-improving effect, it can be set preferably to 20 U or less, more preferably 10 U or less, even more preferably 5 U or less, even more preferably 4 U or less, even more preferably 3 U or less, even more preferably 2 U or less, and even more preferably 1 U or less.

In the present technique, when β-amylase and α-amylase are used in combination, the content ratio is determined based on the above-mentioned contents of each enzyme, but from the viewpoint of further enhancing the effect, the content of β-amylase per 1 U of α-amylase can be set to preferably 0.001 U or more, more preferably 0.01 U or more, even more preferably 0.05 U or more, still more preferably 0.08 U or more, even more preferably 0.1 U or more, and even more preferably 0.12 U or more.

The upper limit of the range of β-amylase content per 1 U of α-amylase can be set, for example, to 100 U or less, and from the viewpoint of further enhancing the shape-retainability-improving effect, it can be set preferably to 20 U or less, more preferably 10 U or less, even more preferably 5.0 U or less, even more preferably 2.0 U or less, even more preferably 1 U or less, even more preferably 0.5 U or less, and even more preferably 0.2 U or less.

(5) Monoglycerides, diglycerides

A monoglyceride and/or a diglyceride can be used for the enzyme preparation for improving the shape-retainability according to the present technique. By using a monoglyceride and/or a diglyceride, the shape-retainability-improving effect of the produced plant-base ice cream can be further enhanced.

As the monoglyceride and/or diglyceride that can be used in the present technique, one or more types of monoglycerides and/or diglycerides that can be used in the food industry can be freely selected and used as long as the effect of the present technique is not impaired. Examples of monoglycerides include glycerin caprylic acid ester, glycerin capric acid ester, glycerin lauric acid ester, glycerin stearate ester, glycerin oleic acid ester, and glycerin behenic acid ester. Examples of diglycerides include those in which two of the same or different fatty acids of these monoglycerides are bonded.

The content of monoglyceride and/or diglyceride in the enzyme preparation for improving the shape-retainability according to the present technique can be freely set as long as it does not impair the effect of the present technique. The content of monoglyceride and/or diglyceride can be set to, for example, 0.5 parts by weight or more relative to 100 parts by weight of plant-base fat used in the plant-base ice cream to be produced, and from the viewpoint of further improving the shape-retainability, it can be set to preferably 1 part by weight or more, more preferably 2 parts by weight or more.

The upper limit of the content of monoglycerides and/or diglycerides in the enzyme preparation for improving the shape-retainability according to the present technique is not particularly limited as long as it does not impair the effects of the present technique, but can be set, for example, to 20 parts by weight or less, preferably 10 parts by weight or less, and more preferably 8 parts by weight or less, per 100 parts by weight of plant-base fat used in the plant-base ice cream to be produced.

(6) Other ingredients

When the enzyme preparation for improving the shape-retainability according to the present technique contains one or more enzymes selected from the group consisting of the maltotriose-producing amylase, lipase, and β-amylase described above, the enzyme preparation may be composed only of one or more enzymes selected from the group consisting of the maltotriose-producing amylase, lipase, and β-amylase described above, may use the above-mentioned α-amylase, monoglyceride, diglyceride, or the like together, and may contain one or more other components selected freely, as long as the effects of the present technique are not impaired. Examples of other components that can be used include excipients, pH adjusters, colorants, flavoring agents, disintegrants, lubricants, stabilizers, and other components that are normally used in formulations. Furthermore, components having known or future functions can be used in combination as appropriate depending on the purpose.

2. Plant-base ice cream

The plant-base ice cream according to the present technique is a plant-base ice cream produced using the enzyme preparation for improving the shape-retainability according to the present technique described above. The plant-base ice cream according to the present technique is characterized in that in the production process, the plant-base material is treated with one or more enzymes selected from the group consisting of maltotriose-producing amylase, lipase, and β-amylase, thereby increasing the content of one or more selected from maltotriose, monoglyceride, and diglyceride, and thus it has higher shape-retainability, higher stability during storage, and is less likely to melt than a plant-base ice cream produced without using the enzyme preparation for improving the shape-retainability according to the present technique. That is, the plant-base ice cream according to the present technique is characterized in that it contains one or more materials selected from a carbohydrate-containing plant-base material treated with maltotriose-producing amylase, a fat-containing plant-base material treated with lipase, and a carbohydrate-containing plant-base material treated with β-amylase.

The content of maltotriose contained in the plant-base ice cream according to the present technique can be, for example, 10 parts by weight or more, preferably 15 parts by weight or more, more preferably 20 parts by weight or more, and even more preferably 30 parts by weight or more, per 100 parts by weight of carbohydrates derived from carbohydrate-containing plant-base materials.

The upper limit of the maltotriose content in the plant-base ice cream according to the present technique can be, for example, 60 parts by weight or less, preferably 50 parts by weight or less, more preferably 45 parts by weight or less, even more preferably 40 parts by weight or less, and even more preferably 35 parts by weight or less, per 100 parts by weight of carbohydrates derived from carbohydrate-containing plant-base materials.

The content of maltotriose contained in the plant-base ice cream according to the present technique is, for example, 0.1 parts by weight or more, preferably 0.3 parts by weight or more, more preferably 1 part by weight or more, and even more preferably 1.5 parts by weight or more, per 100 parts by weight of a plant-base ice cream.

The upper limit of the maltotriose content contained in the plant-base ice cream according to the present technique is, for example, 10 parts by weight or less, preferably 5 parts by weight or less, more preferably 4 parts by weight or less, even more preferably 3 parts by weight or less, and even more preferably 2.5 parts by weight or less, per 100 parts by weight of a plant-base ice cream.

The content of monoglycerides and/or diglycerides contained in the plant-base ice cream of the present invention can be, for example, 1 part by weight or more, preferably 2 parts by weight or more, more preferably 5 parts by weight or more, and even more preferably 6 parts by weight or more, per 100 parts by weight of a fat.

In order to prevent a deterioration in flavor due to an increase in the amount of released saturated fatty acids, the upper limit of the content of monoglycerides and/or diglycerides contained in the plant-base ice cream according to the present technique can be, for example, 70 parts by weight or less, 50 parts by weight or less, 30 parts by weight or less, 20 parts by weight or less, 15 parts by weight or less, or 10 parts by mass or less, per 100 parts by weight of a fat.

The content of monoglycerides and/or diglycerides contained in the plant-base ice cream of the present invention is, for example, 0.1 parts by weight or more, preferably 0.2 parts by weight or more, more preferably 0.3 parts by weight or more, and even more preferably 0.35 parts by weight or more, per 100 parts by weight of the plant-base ice cream.

In order to prevent a deterioration in flavor due to an increase in the amount of released saturated fatty acids, the upper limit of the content of monoglycerides and/or diglycerides contained in the plant-base ice cream according to the present technique can be, for example, 5 parts by weight or less, 3 parts by weight or less, 2 parts by weight or less, 1 part by weight or less, 0.8 parts by weight or less, or 0.5 parts by weight or less per 100 parts by weight of the plant-base ice cream.

3. Method for producing a plant-base ice cream and method for improving the shape-retainability

The method for producing a plant-base food or drink and the method for improving the shape-retainability according to the present technique are methods that at least include one or more steps selected from a maltotriose-producing amylase action step in which maltotriose-producing amylase is allowed to act on a carbohydrate-containing plant-base material, a lipase action step in which lipase is allowed to act on a fat-containing plant-base material, and a β-amylase action step in which β-amylase is allowed to act on a carbohydrate-containing plant-base material. In addition, an α-amylase action step in which α-amylase is allowed to act on a carbohydrate-containing plant-base material, a monoglyceride and/or diglyceride addition step in which monoglyceride and/or diglyceride are added, a recovery step, etc. may also be performed. In addition, depending on the type of plant-base ice cream, etc., a general plant-base ice cream production process may be performed before, after, or simultaneously with each step, as long as the effect of the present technique is not impaired. Each step, etc. will be described in detail below.

(1) Materials

As the plant-base materials used in the method for producing a plant-base food or drink and the method for improving the shape-retainability according to the present technique, a carbohydrate-containing plant-base material and a fat-containing plant-base material are used.

(1-1) Carbohydrate-containing plant-base material

The carbohydrate-containing plant-base material that can be used in the present technique is not particularly limited as long as it contains carbohydrates derived from plants, but a typical example is a material obtained by mechanically crushing grains (e.g., oats) using a mill, etc. The carbohydrate preferably contains polysaccharides (e.g., starch, dextrin, etc.).
The carbohydrate-containing plant-base material and the fat-containing plant-base material described below may be different materials or may be the same material.
The form of the carbohydrate-containing plant-base material is not limited as long as it does not impair the effects of the present technique, and examples of the form include powder, flakes, and granules.
The content of carbohydrates (preferably starch) contained in the carbohydrate-containing plant-base material (based on the weight of the carbohydrate-containing plant-base material in a dry state) is not particularly limited as long as it does not impair the effects of the present technique, and can be, for example, 20% by weight or more, and from the viewpoint of further improving shape-retainability, the content is preferably 50% by weight or more, 55% by weight or more, more preferably 60% by weight or more, and even more preferably 65% by weight or more.
The upper limit of the carbohydrate (preferably starch) content contained in the carbohydrate-containing plant-base material is not particularly limited as long as it does not impair the effects of the present technique, and can be, for example, 95% by weight or less, preferably 90% by weight or less, more preferably 85% by weight or less, and even more preferably 80% by weight or less.
The origin of the plant-base carbohydrate is not particularly limited as long as it does not impair the effect of the present technique, and examples thereof include cereals such as oats, barley (non-glutinous barley, glutinous barley), rice, wheat, rye, buckwheat, barnyard millet, millet, teff, quinoa, and corn; pulses such as adzuki beans, broad beans, peas, chickpeas, mung beans, lupine beans, and kidney beans; nuts and seeds such as almonds, cashew nuts, hazelnuts, pecan nuts, macadamia nuts, pistachios, walnuts, Brazil nuts, peanuts, coconuts, pili nuts, chestnuts, sesame seeds, industrial hemp seeds, canary seeds, linseeds, and pine nuts; and algae, and one type of these plant-base carbohydrates may be used alone, or multiple types thereof may be used in combination. Among these, in the present technique, it is particularly preferable to use a material containing carbohydrates derived from cereals, and it is more preferable to use a material containing carbohydrates derived from oats.

(1-2) Fat-containing plant-base materials

The fat-containing plant-base material that can be used in the present technique is not particularly limited as long as it contains a fat derived from a plant, but typical examples include a fat obtained by physically squeezing or extracting with a solvent from a plant-base raw material, or a combination thereof, and then undergoing various refining processes.
The form of the fat-containing plant-base material is not limited as long as it does not impair the effects of the present technique, and examples of the form include solid and liquid.
The content of a fat contained in the fat-containing plant-base material (based on the weight of the fat-containing plant-base material used) is not particularly limited as long as it does not impair the effects of the present technique, but can be, for example, 20% by weight or more, and from the viewpoint of further improving shape-retainability, preferably 60% by w eight or more, more preferably 80% by weight or more, and even more preferably 95% by weight or more.
The upper limit of the content of the fat contained in the fat-containing plant-base material is not particularly limited as long as it does not impair the effects of the present technique, and examples of the upper limit include 99% by weight or less, 97% by weight or less, and 95% by weight or less.
The origin of the plant-base fat is not particularly limited as long as it does not impair the effects of the present technique, and examples thereof include coconut oil, soybean oil, rapeseed oil, rice oil, corn oil, sunflower oil, cottonseed oil, peanut oil, safflower oil, olive oil, palm oil, palm kernel oil, Palmae oil, cocoa butter, etc., and one type of these plant-base fats may be used alone or multiple types may be used in combination. Among these, in the present technique, it is particularly preferable to use a material containing coconut oil.

(2) Maltotriose-producing amylase action step

The maltotriose-producing amylase action step is a step of allowing the maltotriose-producing amylase to act on the carbohydrate-containing plant-base material. The amount of maltotriose-producing amylase added in the maltotriose-producing amylase action step can be freely set as long as it does not impair the effect of the present technique. The specific amount of maltotriose-producing amylase added in the present technique is the same as the content of maltotriose-producing amylase in the above-mentioned enzyme preparation for improving the shape-retainability, so a description thereof will be omitted here.

Various conditions for the maltotriose-producing amylase action step can be freely set as long as they do not impair the effects of the present technique. For example, the pH, temperature, action time, etc. can be set according to the physicochemical properties of the maltotriose-producing amylase used, such as the optimum pH, stable pH range, optimum temperature, and temperature stability. The pH can be set, for example, to pH 5 to 9, preferably pH 6 to 8, and more preferably pH 6.5 to 7.5. The temperature can be set, for example, to 20 to 80°C, preferably 30 to 70°C, and more preferably 40 to 60°C. The action time can be set, for example, to 5 minutes to 24 hours, preferably 15 minutes to 12 hours, and more preferably 30 minutes to 2 hours. The optimal reaction conditions can be determined through preliminary experiments.

In the carbohydrate-containing plant-base material that has been subjected to the maltotriose-producing amylase action step, the content of maltotriose is increased by the action of the maltotriose-producing amylase. The content of maltotriose contained in the carbohydrate-containing plant-base material that has been subjected to the maltotriose-producing amylase action step (hereinafter also referred to as "carbohydrate-containing plant-base material treated with maltotriose-producing amylase") is, for example, 10 parts by weight or more per 100 parts by weight of carbohydrate. Preferably, the content is 15 parts by weight or more, more preferably 20 parts by weight or more, and even more preferably 30 parts by weight or more.

The upper limit of the maltotriose content in the carbohydrate-containing plant-base material that has been subjected to the maltotriose-producing amylase action step is, for example, 60 parts by weight or less per 100 parts by weight of carbohydrate. It is preferably 50 parts by weight or less, more preferably 45 parts by weight or less, even more preferably 40 parts by weight or less, and even more preferably 35 parts by weight or less.

(3) Lipase action step

The lipase action step is a step of allowing lipase to act on a fat-containing plant-base material. The amount of lipase added in the lipase action step can be freely set as long as it does not impair the effect of the present technique. The specific amount of lipase added in the present technique is the same as the content of lipase in the enzyme preparation for improving the shape-retainability described above, so the explanation is omitted here.

Various conditions for the lipase action step can be freely set as long as they do not impair the effects of the present technique. For example, the pH, temperature, action time, etc. can be set according to the physicochemical properties of the lipase used, such as the optimum pH, stable pH range, optimum temperature, and temperature stability. The pH can be set, for example, to pH 5 to 9, preferably pH 6 to 8, and more preferably pH 6.5 to 7.5. The temperature can be set, for example, to 20 to 80°C, preferably 30 to 70°C, and more preferably 40 to 60°C. The action time can be set, for example, to 5 minutes to 24 hours, preferably 10 minutes to 12 hours, and more preferably 20 minutes to 1 hour. The optimal reaction conditions can be determined through preliminary experiments.

In the fat-containing plant-base material that has been subjected to the lipase action step, the content of monoglycerides and/or diglycerides is increased by the action of lipase. The content of monoglycerides and/or diglycerides contained in the fat-containing plant-base material that has been subjected to the lipase action step (hereinafter also referred to as "lipase-treated fat-containing plant-base material") is, for example, 0.6 parts by weight or more, preferably 0.7 parts by weight or more, more preferably 0.8 parts by weight or more, even more preferably 0.9 parts by weight or more, and even more preferably 1 part by weight or more, per 100 parts by weight of a fat.

In addition, the upper limit of the content of monoglycerides and/or diglycerides contained in the fat-containing plant-base material that has been subjected to the lipase action step can be, for example, 10 parts by weight or less, 8 parts by weight or less, 6 parts by weight or less, 4 parts by weight or less, 3 parts by weight or less, or 2 parts by weight or less per 100 parts by weight of the fat.

The fat-containing plant-base material that has been subjected to the lipase action step has an increased acid value due to the action of lipase. The acid value of the fat-containing plant-base material that has been subjected to the lipase action step is, for example, 1.1 or more, preferably 1.2 or more, more preferably 1.3 or more, even more preferably 1.5 or more, and even more preferably 1.7 or more.

The upper limit of the acid value contained in the fat-containing plant-base material that has been subjected to the lipase action step is, for example, 5 or less, preferably 4 or less, more preferably 3 or less, and even more preferably 2 or less.

Either the maltotriose-producing amylase action step or the lipase action step may be performed, or both may be performed. When both are performed, the order of the maltotriose-producing amylase action step and the lipase action step is not particularly limited. For example, each step is performed separately, and a carbohydrate-containing plant-base material that has been subjected to the maltotriose-producing amylase action step is mixed with a fat-containing plant-base material that has been subjected to the lipase action step, thereby making it possible to produce a plant-base ice cream. In addition, examples of the method include a method of allowing a material containing plant-base carbohydrate and plant-base fat to act with maltotriose-producing amylase and then allowing lipase to act thereon, a method of allowing a material containing plant-base carbohydrate and plant-base fat to act with lipase and then allowing maltotriose-producing amylase to act thereon, and a method of allowing a material containing plant-base carbohydrate and plant-base fat to act simultaneously with maltotriose-producing amylase and lipase. In the present technique, it is particularly preferable to adopt a method of producing a plant-base ice cream by carrying out each step separately and mixing a carbohydrate-containing plant-base material that has been subjected to a maltotriose-producing amylase action step with a fat-containing plant-base material that has been subjected to a lipase action step.

(4) β-Amylase action step

The β-amylase action step is a step in which β-amylase is allowed to act on the carbohydrate-containing plant-base material. The amount of β-amylase added in the β-amylase action step can be freely set as long as it does not impair the effect of the present technique. The specific amount of β-amylase added in the present technique is the same as the content of β-amylase in the above-mentioned enzyme preparation for improving the shape-retainability, so a description thereof will be omitted here.

Various conditions for the β-amylase action step can be freely set as long as they do not impair the effects of the present technique. For example, the pH, temperature, action time, etc. can be set according to the physicochemical properties of the β-amylase used, such as the optimum pH, stable pH range, optimum temperature, and temperature stability. The pH can be set, for example, to pH 5 to 9, preferably pH 6 to 8, and more preferably pH 6.5 to 7.5. The temperature can be set, for example, to 20 to 80°C, preferably 30 to 70°C, and more preferably 40 to 60°C. The action time can be set, for example, to 5 minutes to 24 hours, preferably 15 minutes to 12 hours, and more preferably 30 minutes to 2 hours. The optimal reaction conditions can be determined through preliminary experiments.

(5) α-Amylase action step

The α-amylase action step is a step in which α-amylase is allowed to act on a carbohydrate-containing plant-base material. By allowing α-amylase to act on the carbohydrate-containing plant-base material, the starch in the carbohydrate-containing plant-base material can be liquefied. In the present technique, the α-amylase action step is not an essential step, and for example, by using a carbohydrate-containing plant-base material that has been previously treated with α-amylase, the same effect as that achieved by carrying out the α-amylase action step can be obtained.

The amount of α-amylase added in the α-amylase action step can be freely set as long as it does not impair the effect of the present technique. The specific amount of α-amylase added in the present technique is the same as the content of α-amylase in the above-mentioned enzyme preparation for improving the shape-retainability, so the explanation will be omitted here.

Various conditions for the α-amylase action step can be freely set as long as they do not impair the effects of the present technique. For example, the pH, temperature, action time, etc. can be set according to the physicochemical properties of the α-amylase used, such as the optimum pH, stable pH range, optimum temperature, and temperature stability. The pH can be set, for example, to pH 5.0 to 8.0, preferably pH 5.5 to 7.5, and more preferably pH 6.0 to 7.0. The temperature can be set, for example, to 30°C to 80°C, preferably 40°C to 75°C, and more preferably 50°C to 70°C. The action time can be set, for example, to 10 minutes to 12 hours, preferably 30 minutes to 6 hours, and more preferably 1 hour to 3 hours. The optimal reaction conditions can be determined through preliminary experiments.

The order in which the α-amylase action step is performed is not limited as long as it does not impair the effects of the present technique, but it is preferable to perform the α-amylase action step before the maltotriose-producing amylase action step and/or the β-amylase action step, or simultaneously with the maltotriose-producing amylase action step and/or the β-amylase action step.

After each of the enzyme action steps described above, an enzyme inactivation step can also be carried out.

(6) Monoglyceride and/or diglyceride addition step

The monoglyceride and/or diglyceride adding step is a step of adding monoglyceride and/or diglyceride in the manufacturing process of a plant-base ice cream. In the present technique, the monoglyceride and/or diglyceride adding step is not an essential step, and for example, by using a plant-base material containing monoglyceride and/or diglyceride, the same effect as that of the monoglyceride and/or diglyceride adding step can be obtained.

The amount of monoglyceride and/or diglyceride added in the monoglyceride and/or diglyceride adding step can be freely set as long as it does not impair the effect of the present technique. The specific amount of monoglyceride and/or diglyceride added in the present technique is the same as the content of monoglyceride and/or diglyceride in the above-mentioned enzyme preparation for improving the shape-retainability, so a description thereof will be omitted here.

The order of performing the monoglyceride and/or diglyceride addition step is not limited as long as it does not impair the effect of the present technique. For example, it can be freely selected from a method of performing a maltotriose-producing amylase action step and/or a lipase action step after adding a monoglyceride and/or a diglyceride to a carbohydrate-containing plant-base material and/or a fat-containing plant-base material, a method of adding a monoglyceride and/or a diglyceride to a carbohydrate-containing plant-base material and/or a fat-containing plant-base material that has been subjected to a maltotriose-producing amylase action step and/or a lipase action step, etc. In the present technique, it is particularly preferable to adopt a method of performing a lipase action step after adding a monoglyceride and/or a diglyceride to a fat-containing plant-base material.

(7) Recovery step

The recovery step is a step of recovering the plant-base ice cream produced through one or more steps selected from a maltotriose-producing amylase action step, a lipase action step, and a β-amylase action step, and, if necessary, an α-amylase action step, a monoglyceride and/or diglyceride addition step, etc. As for the specific recovery method, one or a combination of two or more general recovery methods used in the production of a plant-base ice cream can be used freely depending on the type and form of the plant-base ice cream to be produced.

The method for producing a plant-base ice cream and the method for improving the shape-retainability according to the present technique described above can be embodied as including the following steps (1) to (8).

(1) A step of preparing a carbohydrate-containing plant-base material and/or a fat-containing plant-base material,
(2) A step of allowing maltotriose-producing amylase to act on the prepared carbohydrate-containing plant-base material,
(3) A step of allowing lipase to act on the prepared fat-containing plant-base material,
(4) A step of allowing β-amylase to act on the prepared carbohydrate-containing plant-base material,
(5) A step of allowing α-amylase to act on the prepared carbohydrate-containing plant-base material,
(6) A step of adding monoglyceride and/or diglyceride,
(7) A step of inactivating the enzyme,
(8) A step of recovering the produced plant-base ice cream.

Any one of steps (2) to (4) may be performed, or two or all of the steps may be performed. When two or more steps are performed, the order is not particularly limited, and they may be performed separately or simultaneously. When performing the steps separately, the enzyme in step (7) may be inactivated between each step as necessary. Furthermore, steps (5) to (8) are not essential and may be performed as necessary. When performing step (5), the order of steps (2) to (4) is not particularly limited, and they may be performed separately or simultaneously. When performing the steps separately, the enzyme in step (7) may be inactivated between each step as necessary. Furthermore, when performing step (6), it may be performed between any of steps (1) to (7) or simultaneously with any of steps (2) to (5).

EXAMPLES

The present invention will be described in more detail below with reference to examples. Note that the examples described below are representative examples of the present invention, and the scope of the present invention should not be construed as being narrowed by these examples.

1. Materials used, enzymes used

The materials and enzymes used in the examples are shown in Table 1 below.

[Table 1]

| Material, enzyme | | Product name, origin, etc. | Manufacturer |
|---|---|---|---|
| Carbohydrate-containing plant-base material | Oat | OAT FLOUR (starch content: 67 wt%) | Pure Supplements |

(continued)

| Material, enzyme | | Product name, origin, etc. | Manufacturer |
|---|---|---|---|
| Fat-containing plant-base material | Coconut oil | Refined COCONUT OIL | Nutiva |
| Monoglyceride, diglyceride | | Mono and Diglyceride Flakes | Modernist pantry |
| Others | Glucose | Dextrose | EARTHBORN Elements |
| | Sugar | Sugar | United Sugars Corporation |
| | Vanilla essence | Pure Vanilla Extract | McCormick |
| | Salt | Ionized salt | MORTON |
| Enzyme | Maltotriose-producing amylase | Microbacterium sp. organism-derived | Amano Enzyme Inc. |
| | Lipase | Penicillium roqueforti organism-derived | Amano Enzyme Inc. |
| | β-amylase | Bacillus flexus organism-derived | Amano Enzyme Inc. |
| | α-amylase | Bacillus amyloliquefaciens organism-derived | Amano Enzyme Inc. |

2. Enzyme activity measurement method

[Method for measuring maltotriose-producing amylase activity]

An appropriate amount of enzyme was added to 0.5 ml of 2% soluble starch dissolved in 0.1 M phosphate buffer (pH 7.0), and the reaction was carried out at 40°C in a total volume of 1.0 ml, and the amount of maltotriose and other reducing sugars produced was quantified by the Somogyi-Nelson method. The amount of enzyme that produces reducing sugars equivalent to 1 μmole of glucose per minute under this condition was defined as 1 unit (1 U).

[Method for measuring lipase activity]

First, 45 g of olive oil was mixed with 150 mL of an emulsion (18.5 g/L polyvinyl alcohol (completely saponified, saponification degree $98.8 \pm 0.2$), 1.5g/L polyvinyl alcohol (partially saponified, saponification degree $88.8 \pm 1.0$)) and emulsified using a homogenizer to prepare a substrate solution. One milliliter (1 mL) of a lipase enzyme solution was added to 5 mL of the prepared substrate solution and 4 mL of 0.1 mol/L phosphate buffer (pH 7.0) and reacted at 37°C, and after 30 minutes, 10 mL of an ethanol/acetone mixture was added to stop the enzyme reaction. Next, 10 mL of a 0.05 mol/L sodium hydroxide solution and 10 mL of an ethanol/acetone mixture were added, and the pH was titrated to 10 with 0.05 mol/L hydrochloric acid. The amount of enzyme that causes an increase of 1 μmol of fatty acid per minute was defined as 1 unit (1 U).

[Method for measuring β-amylase activity]

The measurement was performed according to the method described in the Japan's Specifications and Standards for Food Additives (9th edition). Specifically, potato starch was used as a substrate, and it was dried at 105°C for 2 hours in advance, and 1.0 g of the dried product thereof was weighed out, 20 mL of water was added, and 5 mL of sodium hydroxide test solution (2 mol/L) was gradually added while stirring to form a paste. Next, the mixture was heated in a water bath while stirring for 3 minutes, and then 25 mL of water was added. After cooling, the mixture was neutralized by adding hydrochloric acid test solution (2 mol/L) and hydrochloric acid test solution (0.1 mol/L), and 10 mL of 1 mol/L acetic acid-sodium acetate buffer solution (pH 5.0) was added, and water was further added to make 100 mL to prepare a substrate solution. Ten milliliters (10 mL) of the substrate solution was measured, heated at 37°C for 10 minutes, 1 mL of sample solution was added, and immediately shaken, and after heating at the same temperature for 10 or 30 minutes, 4 mL of Fehling's test solution was added, gently shaken, heated in a boiling water bath for 15 minutes, cooled to 25°C or lower, and 2 mL of 30% potassium iodide solution and 2 mL of sulfuric acid (1→6) were added to prepare the test solution. Fehling's test solution was prepared just before use by mixing a copper solution obtained by weighing out 34.66 g of fine crystals of copper(II) sulfate pentahydrate, adding water to dissolve, and making a total volume of 500 mL, and an alkaline tartrate solution obtained by weighing out 173 g of (+)-sodium potassium tartrate tetrahydrate and 50 g of sodium hydroxide, adding water to dissolve, and making a total volume of 500 mL, at a ratio of 1 volume of the copper solution to 1 volume of the alkaline tartrate solution. Separately, 10 mL of water was used instead of the substrate solution, and the procedure was repeated in the same manner as for preparation of the test solution to prepare a control solution. The liberated iodine in the test solution and the control solution was titrated with 0.05 mol/L sodium thiosulfate solution. The end point was determined when the blue color disappeared after adding 1-2 drops of soluble starch test solution when the titration was nearing the end point. The amount of enzyme that increases the reducing power equivalent to 1 mg of glucose per minute was defined as 1 unit (1 U), and was calculated from the following formula.

$$\beta\text{-amylase activity (U/g, U/mL)} = \text{amount of glucose (mg)} \times 1/10 \times 1/M$$

$$\text{amount of glucose (mg)} = (b\text{-}a) \times 1.6 \times f$$

a: Titration value of enzyme reaction solution (mL)
b: Titration value of blank solution (mL)
1.6: 1 mL of 0.05 mol/L sodium thiosulfate solution is equivalent to 1.6 mg of glucose.
1/10: Unit conversion factor for reaction time (min)
M: Amount of sample in 1 mL of sample solution (g or mL)
f: Factor of 0.05 mol/L sodium thiosulfate solution (for quantitative analysis)

[Method for measuring $\alpha$-amylase activity]

Ten milliliters (10 mL) of 1% potato dextrin was pretreated at 37°C for 15 minutes, and then 1 mL of amylase solution was added and mixed thoroughly immediately. After 10 minutes at 37°C, 1 mL was taken out and mixed with 10 mL of 0.1 M hydrochloric acid, and 0.5 mL was taken out from the solution and mixed with 10 mL of 0.2 mM iodine solution, and the absorbance (wavelength 660 nm) was measured. The amount of enzyme that reduces the coloration due to iodine reaction by 10% in 1 minute was defined as 1 unit (1 U).

3. Experimental example

<Experimental Example 1>

In Experiment 1, the effect of maltotriose-producing amylase and $\beta$-amylase on improving the shape-retainability of a plant-base ice cream was examined.

(1) Production of plant-base ice cream

One hundred twenty grams (120 g) of oat powder was added to 1 L of purified water and mixed for 20 minutes at 50°C using a heating mixer (Thermomix TM6, Vorwerk) at speed 2. The enzymes shown in Table 2 below were then added and stirred for another hour. The mixture was then passed through cheesecloth to separate the solids, which was used as oat base.

Four point four eight grams (4.48 g) of mono- and diglycerides were added to 89.6 g of refined coconut oil and mixed for 20 minutes at 70°C using a heating mixer ("Thermomix TM6", manufactured by Vorwerk) at speed 2. Next, 89.6 g of glucose and 78.4 g of sugar were added and mixed for another 10 minutes to obtain the coconut oil base.

The prepared oat base was added to the prepared coconut oil base, heated to 85°C, and stirred for 15 minutes. The mixture was then transferred to a bowl and emulsified with a hand mixer for 5 minutes, and then stored in a refrigerator overnight. The next day, 2g of vanilla essence and 2 g of salt were added and mixed for 30 to 45 seconds, and a plant-base ice cream was produced by an ice cream maker (NOSTALGIA PRODUCTS, model: PICM20DBL).

(2) Sugar composition analysis

Zero point five milliliters (0.5 mL) of the oat base obtained above was diluted 5-fold with purified water, centrifuged (13,000 rpm, 5 minutes), and the supernatant was taken out and passed through a membrane filter (0.2 $\mu$m) to analyze the sugar composition by high performance liquid chromatography (detector: RID-20A, Shimadzu Corporation). A CK04S (Mitsubishi Chemical Corporation) column was used, and the degree of polymerization of sugars in particular was analyzed. The ratio of each sugar component to the total peak area is shown in Table 2 below.

(3) Shape-retainability evaluation

The produced plant-base ice cream was filled into paper cups, further frozen overnight at -15°C, and molded. Shape-retainability was evaluated by placing the ice cream, after removing the paper cup, on a mesh plate set horizontally on a balance at room temperature, and readings on the balance were taken every 5 minutes for 90 minutes, starting from the point when the ice cream started to melt and the first drop fell on the balance.

The value calculated using the following formula (1) was used as the evaluation value for shape-retainability.

Shape-retainability (%) = {((weight of ice cream at the start) - (weight on the scale after 90 minutes)) ÷ (weight of ice cream at the start)} × 100
(1)

The shape-retainability increase rate was calculated from the obtained evaluation value (shape-retainability (%)) using the following formula (2).

Shape-retainability increase rate (%) = ((shape-retainability of each test section)/(shape-retainability of control)) × 100
(2)

(4) Results

The results are shown in Table 2 below.

[Table 2]

| | | Control | Test section | |
| --- | --- | --- | --- | --- |
| | | 1 | 1 | 2 |
| Addition amount of enzyme (U/g(carbohydrate-containing plant-base material)) | α-amylase | 13.5 | 13.5 | 13.5 |
| | β-amylase | - | 1.95 | - |
| | Maltotriose-producing amylase | - | - | 8.4 |
| Carbohydrate composition (%) in oat base | Glucose (G1) | 2.5 | 1.6 | 2.1 |
| | Maltose (G2) | 7.5 | 38.6 | 12.6 |
| | Maltotriose (G3) | 7.5 | 10.3 | 33 |
| Evaluation | Shape-retainability increase rate (%) | - | 128 | 156 |

(5) Observation

As shown in Table 2, test section 1, in which a plant-base ice cream was produced using β-amylase, and test section 2, in which a plant-base ice cream was produced using maltotriose-producing amylase, showed higher shape-retainability than Control 1, in which a plant-base ice cream was produced without using maltotriose-producing amylase. In particular, the improvement in shape-retainability was remarkable in test section 2, in which a plant-base ice cream was produced using maltotriose-producing amylase.

<Experimental Example 2>

In Experiment 2, the addition amount dependence of maltotriose-producing amylase was examined.

(1) Production of plant-base ice cream

In the same manner as in Experimental Example 1, plant-base ice creams of Control 2, test section 3, and test section 4 shown in Table 3 were produced.

(2) Shape-retainability evaluation

The rate of increase in shape-retainability was evaluated in the same manner as in Experimental Example 1.

(3) Results

The results are shown in Table 3 below.

[Table 3]

|  |  | Control | Test section | |
|---|---|---|---|---|
|  |  | 2 | 3 | 4 |
| Addition amount of enzyme (U/g(carbohydrate-containing plant-base material)) | α-amylase | 13.5 | 13.5 | 13.5 |
|  | Maltotriose-producing amylase | - | 1.2 | 8.4 |
| Evaluation | Shape-retainability increase rate (%) | - | 226 | 216 |

(4) Observation

As shown in Table 3, test sections 3 and 4, in which a plant-base ice cream was produced using maltotriose-producing amylase, showed higher shape-retainability than Control 2, in which a plant-base ice cream was produced without using maltotriose-producing amylase. Furthermore, the results of test sections 3 and 4 showed that even about 1.2 U of maltotriose-producing amylase was able to sufficiently improve shape-retainability.

<Experimental Example 3>

In Experimental Example 3, the effect of lipase on improving the shape-retainability of a plant-base ice cream was investigated.

(1) Production of plant-base ice cream

One hundred twenty grams (120 g) of oat powder was added to 1 L of purified water and mixed using a heating mixer ("Thermomix TM6", manufactured by Vorwerk) at 50°C for 20 minutes at speed 2. Next, α-amylase was added in the amount shown in Table 4 below and stirred for another hour. The mixture was then passed through cheesecloth to separate the solids, which was used as oat base.
Four point four eight grams (4.48 g) of mono- and diglycerides were added to 89.6 g of refined coconut oil, and mixed at 70°C for 20 minutes using a heating mixer ("Thermomix TM6", manufactured by Vorwerk) at speed 2. Next, for test section 5, the amount of lipase shown in Table 4 below was added, and the mixture was further stirred at 50°C for 30 minutes. After that, 89.6 g of glucose and 78.4 g of sugar were added, and the mixture was further mixed for 10 minutes to obtain the coconut oil base.
Using the prepared oat base and coconut oil base, a plant-base ice cream was produced in the same manner as in Experimental Example 1.

(2) Shape-retainability evaluation

The rate of increase in shape-retainability was evaluated in the same manner as in Experimental Example 1.

(3) Results

The results are shown in Table 4 below.

[Table 4]

| | | | Control | Test section |
|---|---|---|---|---|
| | | | 3 | 5 |
| Addition amount of enzyme | (U/g(carbohydrate-containing plant-base material)) | α-amylase | 13.5 | 13.5 |
| | (U/g(fat-containing plant-base material)) | Lipase | - | 2.7 |
| Evaluation | Shape-retainability increase rate (%) | | - | 181 |

(4) Observation

As shown in Table 4, test section 5, in which plant-base ice cream was produced using lipase, showed higher shape-retainability than Control Group 3, in which plant-base ice cream was produced without using lipase.

<Experimental Example 4>

In Experimental Example 4, the addition amount dependence of lipase was examined.

(1) Production of plant-base ice cream

In the same manner as in Experimental Example 3, the plant-base ice creams of Control 4, test sections 6 and 7 shown in Table 5 were produced.

(2) Shape-retainability evaluation

The rate of increase in shape-retainability was evaluated in the same manner as in Experimental Example 1.

(3) Results

The results are shown in Table 5 below.

[Table 5]

| | | | Control | Test section | |
|---|---|---|---|---|---|
| | | | 4 | 6 | 7 |
| Addition amount of enzyme | (U/g(carbohydrate-containing plant-base material)) | α-amylase | 13.5 | 13.5 | 13.5 |
| | (U/g(fat-containing plant-base material)) | Lipase | - | 2.7 | 5.4 |
| Evaluation | Shape-retainability increase rate (%) | | - | 180 | 410 |

(5) Observation

As shown in Table 5, it was found that the shape-retainability was improved by allowing lipase to act on the fat-containing plant-base material. It was also found that the shape-retainability was improved depending on the amount of lipase added.

**Claims**

1. An enzyme preparation for improving the shape-retainability of a plant-base ice cream, comprising one or more enzymes selected from the group consisting of maltotriose-producing amylase, lipase, and β-amylase.

2. A plant-base ice cream, in which the enzyme preparation for improving the shape-retainability as described in claim 1 is used.

3. A method for producing a plant-base ice cream comprising one or more steps selected from

a maltotriose-producing amylase action step in which maltotriose-producing amylase is allowed to act on a carbohydrate-containing plant-base material,
a lipase action step in which lipase is allowed to act on a fat-containing plant-base material, and
a β-amylase action step in which β-amylase is allowed to act on a carbohydrate-containing plant-base material.

4. A method for improving the shape-retainability of a plant-base ice cream comprising one or more steps selected from

a maltotriose-producing amylase action step in which maltotriose-producing amylase is allowed to act on a carbohydrate-containing plant-base material,
a lipase action step in which lipase is allowed to act on a fat-containing plant-base material, and
a β-amylase action step in which β-amylase is allowed to act on a carbohydrate-containing plant-base material.

## INTERNATIONAL SEARCH REPORT

| International application No. |
|---|
| **PCT/JP2023/022885** |

### A. CLASSIFICATION OF SUBJECT MATTER

*A23G 9/36*(2006.01)i; *A23D 9/00*(2006.01)i; *A23G 9/34*(2006.01)i; *A23G 9/42*(2006.01)i; *A23L 9/20*(2016.01)i; *A23L 29/212*(2016.01)i; *C12N 9/20*(2006.01)i; *C12N 9/26*(2006.01)i
FI:    A23G9/36; C12N9/26 A; C12N9/20; A23G9/42; A23G9/34; A23D9/00 512; A23L9/20; A23L29/212

According to International Patent Classification (IPC) or to both national classification and IPC

### B. FIELDS SEARCHED

Minimum documentation searched (classification system followed by classification symbols)

A23G9/36; A23D9/00; A23G9/34; A23G9/42; A23L9/20; A23L29/212; C12N9/20; C12N9/26

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| X | よっちみろ屋ホームページ [online], 25 January 2021 [retrieved on 16 August 2023], Internet: <URL: http://www.yottimiroya.com/?p=190>, non-official translation (Yottimiroya Homepage.)<br>「甘酒アイス」が新登場, non-official translation (The new arrival of "amazake ice cream".) | 2, 3 |
| X | カヤマ醸造所 公式オンラインショップ [online], 06 July 2021 [retrieved on 18 August 2023], Internet: <URL: https://shop.kayamasyuzou.com/blog/2021/07/06/113409>, non-official translation (Kayama Brewing Official Online Shop.)<br>暑い日には甘酒アイスはいかが?!, non-official translation (How about amazake ice cream on a hot day?!) | 2, 3 |
| X | クックパッド [online], 04 August 2021 [retrieved on 18 August 2023], Internet: <URL: https://cookpad.com/recipe/6888588>, (Cookpad.)<br>豆腐と甘酒のアイスクリーム, non-official translation (Tofu and amazake ice cream.) | 2, 3 |

☑ Further documents are listed in the continuation of Box C.     ☑ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **18 August 2023** | **12 September 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**

| International application No. |
| --- |
| **PCT/JP2023/022885** |

### C.    DOCUMENTS CONSIDERED TO BE RELEVANT

| Category* | Citation of document, with indication, where appropriate, of the relevant passages | Relevant to claim No. |
| --- | --- | --- |
| X | クックパッド [online], 13 May 2021 [retrieved on 18 August 2023], Internet: <URL: https://cookpad.com/recipe/6265218>, (Cookpad.)<br>砂糖なしヴィーガンでも大満足の甘酒アイス, non-official translation (Sugar-free and vegan, but still very satisfying amazake ice cream.) | 2, 3 |
| X | クックパッド [online], 23 April 2020 [retrieved on 18 August 2023], Internet: <URL: https://cookpad.com/recipe/6162631>, (Cookpad.)<br>ヴィーガン抹茶アイス (乳製品・卵不使用), non-official translation (Vegan matcha ice cream (dairy-free, egg-free).) | 2, 3 |
| X | クックパッド [online], 27 April 2020 [retrieved on 18 August 2023], Internet: <URL: https://cookpad.com/recipe/6174796>, (Cookpad.)<br>ヴィーガンココアアイス/乳製品・卵不使用, non-official translation (Vegan cocoa ice cream (dairy-free, egg-free).) | 2, 3 |
| X | クックパッド [online], 03 May 2021 [retrieved on 18 August 2023], Internet: <URL: https://cookpad.com/recipe/6763564>, (Cookpad.)<br>ココナッツミルクとバナナの甘酒アイス, non-official translation (Coconut milk and banana amazake ice cream.) | 2, 3 |
| X | クックパッド [online], 17 August 2021 [retrieved on 18 August 2023], Internet: <URL: https://cookpad.com/recipe/6902569>, (Cookpad.)<br>材料は3つ!甘酒と豆乳のアイスクリーム, non-official translation (Only 3 ingredients! amazake and soy milk ice cream.) | 2, 3 |
| X | JP 59-227249 A (HANAMARUKI KK) 20 December 1984 (1984-12-20)<br>claims, examples | 2, 3 |
| X | JP 2010-187592 A (SAN-EI SUCROCHEMICAL CO., LTD.) 02 September 2010 (2010-09-02)<br>claims, paragraph [0024], examples | 1-4 |
| X | JP 5-211852 A (THE NISSHIN OIL MILLS, LTD.) 24 August 1993 (1993-08-24)<br>claims, paragraph [0001], examples | 1-4 |
| A | HAKKO JUICE BAR [online], 05 April 2019 [retrieved on 18 August 2023], Internet: <URL: https://kiroro-pokkuru.com/archives/283/><br>酵素の多い食品は?, non-official translation (What foods are rich in enzymes?) | 1-4 |

Form PCT/ISA/210 (second sheet) (January 2015)

**INTERNATIONAL SEARCH REPORT**
Information on patent family members

International application No.

**PCT/JP2023/022885**

| Patent document cited in search report | | | Publication date (day/month/year) | Patent family member(s) | Publication date (day/month/year) |
|---|---|---|---|---|---|
| JP | 59-227249 | A | 20 December 1984 | (Family: none) | |
| JP | 2010-187592 | A | 02 September 2010 | (Family: none) | |
| JP | 5-211852 | A | 24 August 1993 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20180184684 A
- JP 3251173 A
- WO 2020090734 A

**Non-patent literature cited in the description**

- Japan's Specifications and Standards for Food Additives